# EUROPEAN PATENT SPECIFICATION

(11) **EP 4 055 412 B1**
(45) Date of publication and mention of the grant of the patent: **07.06.2023**
(21) Application number: 20793407.6
(22) Date of filing: 27.10.2020
(51) Int. Cl.: G01S 15/89, G01S 7/52, A61B 8/08, A61B 8/00

(54) **ULTRASOUND TRANSDUCER ARRAY**
ULTRASCHALLWANDLERARRAY
RÉSEAU DE TRANSDUCTEURS À ULTRASONS

(30) Priority: 05.11.2019 EP 19207080
(43) Date of publication of application: 14.09.2022
(73) Proprietor: Koninklijke Philips N.V., 5656 AG Eindhoven (NL)
(72) Inventor: HAMELMANN, Paul, Christoph, 5656 AE Eindhoven (NL); KOLEN, Alexander, Franciscus, 5656 AE Eindhoven (NL)
(74) Representative: Philips Intellectual Property & Standards
(86) International application number: PCT/EP2020/080113
(87) International publication number: WO 2021/089364

(56) References cited:
- US-A1- 2003 135 135
- US-A1- 2010 191 119
- US-A1- 2018 130 457

## Description

### FIELD OF THE INVENTION

The invention relates to an ultrasound transducer array having a plurality of transducer elements.

### BACKGROUND OF THE INVENTION

US 3780725 discloses a fetal heartbeat monitoring system in which a plurality of ultrasonic beams are transmitted at different angles into the uterus of an expectant mother. A plurality of receiving transducers are inclined at different angles for receiving the signal as it is reflected from the fetus and/or uterus walls, the frequency of the reflected signal differing from that of the transmitted signal by an amount corresponding to the rate of the fetal heart motion. A system of rate computation from the resultant Doppler fetal heart motion signal is employed which utilizes frequency domain techniques. The heart rate signal is extracted from the Doppler heart motion signal by means of a diode demodulator and low frequency bandpass filter.

US 2018/130457 discloses an ultrasound array comprising a plurality of ultrasound transducer elements on a carrier, said carrier further carrying an actuator arrangement of a material having an adjustable shape in response to an electromagnetic stimulus, e.g. an electro active polymer or optically responsive polymer.

US 2003/135135 discloses an ultrasonic irradiation apparatus is presented which is capable of easily irradiating, with ultrasonic waves, a wide area (range) of an object having three-dimensionally curved surfaces.

### SUMMARY OF THE INVENTION

It is, inter alia, an object of the invention to provide an improved ultrasound transducer array. The invention is defined by the independent claims. Advantageous embodiments are defined in the dependent claims.

An aspect of the invention provides an ultrasound transducer array having a plurality of transducer elements each having a respective field of view, the ultrasound transducer array being capable of being positioned around an object having a curved surface resulting in a convergence of the respective fields of view of the transducer elements, wherein the transducer elements are arranged, or capable of being arranged, on the ultrasound transducer array in a manner opposite to a curvature of the curved surface so as to counteract the convergence of the respective fields of view. Here, the notion "around" does not necessarily mean "all around"; what is meant is that the transducer array is positioned along the curved surface of the object, e.g. on top of the belly of a pregnant woman, so that the transducer elements point to the inside of the belly for fetal monitoring purposes.

The invention is based on the recognition that in a prior art arrangement, due to the curvature of the maternal abdomen, all individual ultrasound transducer elements are directed into the same region of the maternal abdomen, as the ultrasound wave propagation is only perpendicular to the skin surface. This limits the overall field of view (FOV) of the employed ultrasound transducer array. Also, the ultrasound beams of multiple transducer elements may overlap to a large extent, which might lead to high level of acoustic dose (safety risk). In embodiments of this invention, the transducer elements are embedded into the ultrasound transducer array under a specific tilting angle to compensate for the curvature of the maternal abdomen and creating a large overall FOV. Herein, a full and perfect compensation is not needed, and the notion "counteract" should be understood accordingly; an undercompensation or over-compensation is allowed as it suffices if the respective tilting angles under which the various transducer elements are positioned in the transducer array are such that they reduce the net angles resulting from positioning the transducer array on the curved surface of the object. The notions "opposite angles" and "manner opposite to a curvature of the curved surface" should thus also not be understood as (resulting in) angles having exactly the same absolute values.

These and other aspects of the invention will be apparent from and elucidated with reference to the embodiments described hereinafter.

### BRIEF DESCRIPTION OF THE DRAWINGS

Fig. 1 shows a prior art ultrasound transducer array;
Fig. 2 shows an embodiment of an ultrasound transducer array in accordance with the invention; and
Fig. 3 illustrates azimuth and elevation components of the tilting angle of an individual transducer element in the flexible transducer array; and
Fig. 4 shows a schematic representation of an ultrasound system comprising an ultrasound transducer array and a processor.

### DESCRIPTION OF EMBODIMENTS

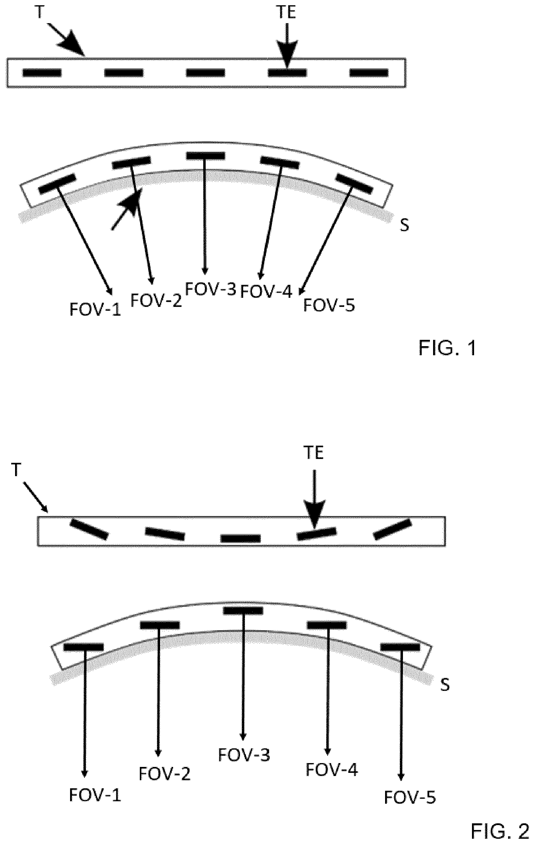

Fig. 1 shows a prior art ultrasound transducer array T having a plurality of transducer elements TE, in this embodiment formed by 5 transducer elements TE, each having a respective field of view FOV-1, FOV-2, FOV-3, FOV-4, FOV-5, schematically illustrated by arrows representing the central axes of the cone-like fields of view. When the ultrasound transducer array T is curved along the skin S of the abdomen of a pregnant woman, as shown in the lower part of Fig. 1, the fields of view FOV-1 - FOV-5 converge so that the cones overlap. As a result, the overall field of view of the ultrasound transducer array T becomes smaller, and where the cones overlap, the ultrasound pressure (acoustic dose) may be relatively high, and even higher than desirable for use with unborn children.

Monitoring the fetal heart rate before and during labor is a standard method to assess the fetal well-being. The most common technology to measure the fetal heart rate is based on Doppler ultrasound. For robust monitoring of the fetal heart rate it is required that the fetal heart is located in the overall field of view FOV of the employed ultrasound transducer. In clinical practice, the fetus moves and, consequently, the fetal heart moves out of the ultrasound beam, leading to long and frequent periods of signal loss. A flexible multi-element ultrasound transducer array T can be wrapped around the maternal abdomen and has the potential to monitor the fetal heart rate continuously for various fetal heart locations, and the possibility of monitoring twins or triplets. A drawback of the flexible ultrasound transducer array T as shown in Fig. 1 is that due to the curvature of the maternal abdomen, all individual transducer elements TE are directed into the same region of the maternal abdomen, as the ultrasound wave propagation is only ultrasound perpendicular to the skin surface. This limits the overall field of view FOV of the employed ultrasound transducer array T. In this invention, we propose to embed the transducer elements TE into the ultrasound transducer array T under specific tilting angles to compensate for the curvature of the maternal abdomen and creating a large overall field of view FOV.

Fig. 2 shows an embodiment of an ultrasound transducer array T in accordance with the invention. Here, in the rest state of the ultrasound transducer array T shown in the upper part of Fig. 2, the various transducer elements TE are pre-tilted in such a way as to counteract the convergence of the fields of view FOV-1, FOV-2, FOV-3, FOV-4, FOV-5 resulting from bending the ultrasound transducer array T around the skin S of the pregnant woman's abdomen as shown in the lower part of Fig. 2. In the idealized situation sketched in Fig. 2, the fields of view FOV-1 - FOV-5 have become perfectly parallel; in reality they still converge or diverge, depending on the relation between the pre-tilting and the curvature of the individual woman's abdomen, the surface of which fluctuates as a result of respiration and/or baby movements, and the claims should be understood accordingly as covering the reality.

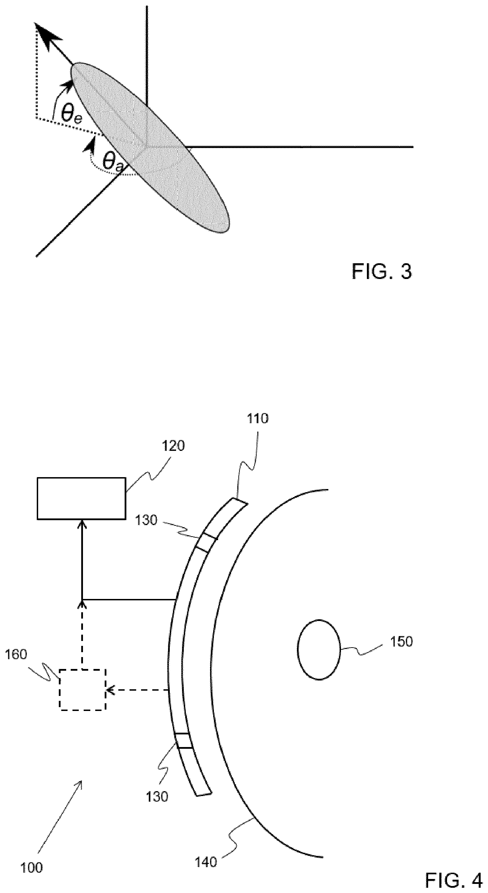

Fig. 3 illustrates azimuth and elevation components of the tilting angle of an individual transducer element in the flexible transducer array. Using two tilting angles allows to compensate for the curvature of the abdomen into two directions. Multiple transducer elements TE may be arranged in e.g. a circular pattern to increase the overall field of view (FOV) of the ultrasound transducer array T.

In embodiments of the present invention, multiple ultrasound transducer elements TE, such as piezoelectric transducer elements (PZTs), are thus embedded in a flexible ultrasound transducer array T. The flexible substrate of the ultrasound transducer array T may be made out of silicone such as Polydimethylsiloxan (PDMS), or any other material which can be made flexible and has suitable acoustic properties. PDMS is a silicone which is flexible and has similar acoustic properties as human tissue and, therefore is used in many biomedical ultrasound applications. To prevent the transducer elements TE from all pointing into the same direction when positioned on the maternal abdomen, the transducer elements TE are embedded into the ultrasound transducer array T with a specific tilting angle θ = [θe θa], with θe and θa being the elevation and azimuth angle, respectively. Tilting the transducer elements TE within the two-dimensional ultrasound transducer array T with an elevation and azimuth angle (see Fig. 3) allows to compensate for the curvature of the abdomen along two directions.

The tilting angle θ should be chosen such that it compensates for the curvature of the maternal abdomen. The curvature of a maternal abdomen depends on the gestational age as well as maternal body mass index (BMI). The maternal abdomen may be considered as a half sphere with radius r, i.e. curvature of x = 1/r. The curvature κ determines the tilting angle θ. For the average maternal abdomen at term, the radius of the sphere lies in the range of r = 25 cm - 40 cm. It should be noted, that it is not essential to tilt the transducer elements TE exactly corresponding to the curvature of the abdomen. Pre-tilting the transducer elements TE will always compensate to some extent for the average curvature of the maternal abdomen and, therefore, always increase/improve the FOV of the ultrasound transducer array T.

In another embodiment, instead of having a one-fit-all solution, different versions of the ultrasound transducer array T can be produced to fit e.g. a small, medium, or a large abdomen. In real-life, the curvature of the abdomen can be determined, e.g. manually by the caregiver or the mother-to-be, or automatically by e.g. camera technology. The best fitting flexible ultrasound transducer array T with the corresponding pre-tilted transducer elements TE for best overall FOV can be selected. In this embodiment, the user would thus be provided with a set of ultrasound transducer arrays T, the set comprising:
a first ultrasound transducer array T having first transducer elements TE arranged in the first ultrasound transducer array T under first respective tilting angles; and
a second ultrasound transducer array T having second transducer elements TE arranged in the second ultrasound transducer array T under second respective tilting angles, each of the second respective tilting angles exceeding a corresponding one of the first respective tilting angles.

In yet another embodiment, the tilting angle θ (in both azimuth and elevation) can be adjusted while the ultrasound transducer array T is positioned on the belly of the pregnant women. This can be implemented by means of a liquid or an ultrasound gel with floating transducer elements T moved by one or more actuators, which may be controlled using e.g. accelerometers, camera, fiber-optic curvature sensors. The position of the floating transducer elements could be fixed if they float in e.g. a sodium acetate liquid that becomes solid when a crystallization nucleus is offered by an actuator (e.g. mechanical, as in ClickHeat heating pads, but alternative actuators like light or temperature are conceivable). In addition, the tilting angle θ can be adjusted continuously over time, to compensate for breathing motion. In such embodiments, the user would thus be provided with an ultrasound transducer array T, wherein respective orientations of the transducer elements TE can be adjusted, and wherein the transducer elements TE are arranged in a medium that can be made to change its state from liquid to solid, thereby fixing the respective orientations.

Fig. 2 illustrates the concept of pre-tilting the transducer elements TE in the flexible array. In prior art Fig. 1, the transducer elements TE are embedded parallel to the transducer array surface, hence, after positioning, all transducer elements TE are directed into the center of the abdomen. By pre-tilting the transducer elements TE, see Fig. 2, after positioning, the transducer elements TE are not parallel to the skin surface which allows insonification of a larger field of view, compared to the situation without pre-tilted transducer elements TE, thereby allowing fetal heart rate monitoring to be robust against location change of the fetal heart.

A further advantage of using pre-tilted transducer elements TE, is that the respective ultrasound beams do not overlap. This reduces the occurrence of destructive and/or constructive interference and/or the accumulation of multiple ultrasound beams in time. Further, having multiple transducer elements TE aiming at the same location does not necessarily improve the estimation of fetal heart rate. Pre-tilting the transducer elements TE enables coverage of a large measurement volume with a reduced number of transducer elements TE, eventually reducing the costs of the system.

In another embodiment of the invention, the ultrasound transducer array T with pre-tilted transducer elements TE allows to generate a FOV large enough to insonify the whole fetus, allowing to monitor fetal movements, such as fetal limb movement.

Advantageously, the transducer elements TE are capacitive micro-manufactured ultrasound transducer (CMUT).

Fig. 4 shows a schematic representation of an ultrasound system 100 comprising an ultrasound transducer array 110 (as described above) and a processor 120. The ultrasound transducer array 110 comprises a plurality of transducer elements 130 (corresponding to the above transducer elements TE) and is adapted to conform to a subject's body 140. At least two ultrasound transducer elements of the plurality of transducer elements 130 are adapted to acquire a plurality of ultrasound signals from a region of interest 150 at different orientations relative to the region of interest. Each individual transducer element 130 is adapted to transmit and receive ultrasound waves. The transducer elements 130 may comprise piezoelectric transducers or CMUT cells.

The transducer array 110 may be adapted to conform to a subject's body 140 in a number of ways. For example, the plurality of transducer elements 130 may be embedded into a flexible silicone layer.

In other words, the transducer array 110 may be adapted to conform to the body 140 of a subject to ensure that the transducer elements have good contact with the body surface. Further, the material layer positioned underneath the transducer elements 130, between the transducer elements 130 and the subject, may be selected to have an appropriate acoustic impedance suitable for ultrasound propagation. The transducer array 110 may be made out of any suitable material, for example, by integrating the transducer elements 130 into a fabric or a belt, which could be wrapped around the subject's body.

In addition, the flexible array does not need to be fully closed. For example, the individual elements could be interconnected by any flexible connector piece, which defines the approximate position of the elements with respect to each other.

Alternatively, the individual transducer elements 130 can be attached directly on the skin of the subject in a similar manner to electrocardiogram (ECG) measurement electrodes directly attaching to the skin.

Further, a transducer element 130 may be formed by a transducer sub-array of seven elements placed on a rigid plate, which may then be positioned on the skin. In this manner. multiple of these sub-arrays may be used to cover a large area while also following the curvature of the measurement subject.

In the example shown in Fig. 4, the ultrasound system 100 is employed to measure a fetal heartbeat. More specifically, the transducer array 110 is positioned adjacent a maternal abdomen in order to insonify a fetal region.

The ultrasound transducer array 110 may further comprise a sensor 160, in which case the processor 120 is adapted to determine a curvature of the ultrasound transducer array 110 based on an output of the sensor 160. The sensor 160 may comprise one or more of: a strain gauge; an accelerometer; a piezoelectric sensor; and a camera. For example, a camera may be used to determine the curvature of the array and, in addition, also the position of the array on the maternal abdomen.

The processor 120 is adapted to receive and process the ultrasound echo signals acquired by the ultrasound transducer array 110.

The present invention, wherein the transducer elements (TE) are arranged, or capable of being arranged, on the ultrasound transducer array (T) in a manner opposite to a curvature of the curved surface (S) so as to counteract the convergence of the respective fields of view, is advantageously used to modify the system of our earlier application PCT/EP2019/060648.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. Measures recited in mutually different dependent claims may advantageously be used in combination.

## Claims

1. An ultrasound transducer array (T) having a plurality of transducer elements (TE) each having a respective field of view (FOV-1 - FOV-5), the ultrasound transducer array (T) being capable of being positioned around an object having a curved surface (S) resulting in a convergence of the respective fields of view of the transducer elements (TE), wherein the transducer elements (TE) are arranged, or capable of being arranged, on the ultrasound transducer array (T) in a manner opposite to a curvature of the curved surface (S) so as to counteract the convergence of the respective fields of view, wherein respective orientations of the transducer elements (TE) can be adjusted, and
**characterized in that**
the transducer elements (TE) are arranged in a medium that can be made to change its state from liquid to solid, thereby fixing the respective orientations.

2. An ultrasound transducer array (T) as claimed in claim 1, wherein the transducer elements (TE) are arranged in the ultrasound transducer array (T) under respective tilting angles opposite to angles at which the transducer elements (TE) will be positioned when the transducer array (T) is positioned around the object.

3. A set of ultrasound transducer arrays (T) as claimed in claim 2, the set comprising:
a first ultrasound transducer array (T) having first transducer elements (TE) arranged in the first ultrasound transducer array (T) under first respective tilting angles; and
a second ultrasound transducer array (T) having second transducer elements (TE) arranged in the second ultrasound transducer array (T) under second respective tilting angles, each of the second respective tilting angles exceeding a corresponding one of the first respective tilting angles.

4. An ultrasound system (100), the system comprising:
an ultrasound transducer array (110) as claimed in any of the preceding claims 1-2, or a set of ultrasound transducer arrays as claimed in claim 3; and
a processor (120) adapted to receive and process ultrasound signals acquired by the ultrasound transducer array (110) or a selected ultrasound transducer array from the set of transducer arrays.

## Patentansprüche

1. Ultraschall-Transducer-Array (T), das eine Vielzahl von Transducerelementen (TE) aufweist, die ein jeweiliges Sichtfeld (FOV-1-FOV-5) aufweisen, wobei das Ultraschall-Transducer-Array (T) in der Lage ist, um ein Objekt positioniert zu werden, das eine gekrümmte Oberfläche (S) aufweist, was eine Konvergenz jeweiliger Sichtfelder der Transducerelemente (TE) ergibt, wobei die Transducerelemente (TE) eingerichtet sind oder in der Lage sind, an dem Ultraschall-Transducer-Array (T) in einer Weise entgegengesetzt zu einer Krümmung der gekrümmten Oberfläche (S) eingerichtet zu werden, um der Konvergenz der jeweiligen Sichtfelder entgegenzuwirken, wobei jeweilige Ausrichtungen der Transducerelemente (TE) eingestellt werden können, und **dadurch gekennzeichnet, dass** die Transducerelemente (TE) in einen Medium eingerichtet sind, das veranlasst werden kann, seinen Zustand von flüssig zu fest zu ändern, wodurch die jeweiligen Ausrichtungen fixiert werden.

2. Ultraschall-Transducer-Array (T) nach Anspruch 1, wobei die Transducerelemente (TE) in dem Ultraschall-Transducer-Array (T) unter jeweiligen Kippwinkeln entgegengesetzt zu Winkeln eingerichtet sind, an welchen die Transducerelemente (TE) positioniert werden, wenn das Transducer-Array (T) um das Objekt positioniert wird.

3. Satz von Ultraschall-Transducer-Arrays (T) nach Anspruch 2, wobei der Satz Folgendes umfasst:
ein erstes Ultraschall-Transducer-Array (T), das erste Transducerelemente (TE) aufweist, die in dem ersten Ultraschall-Transducer-Array (T) mit den ersten jeweiligen Kippwinkeln eingerichtet sind; und
ein zweites Ultraschall-Transducer-Array (T), das zweite Transducerelemente (TE) aufweist, die in dem zweiten Ultraschall-Transducer-Array (T) mit den ersten jeweiligen Kippwinkeln eingerichtet sind, wobei jeder der jeweiligen zweiten Kippwinkel einen entsprechenden der jeweiligen ersten Kippwinkel überschreitet.

4. Ultraschallsystem (100), wobei das System Folgendes umfasst:
ein Ultraschall-Transducer-Array (110) nach einem der vorstehenden Ansprüche 1-2, oder einen Satz von Ultraschall-Transducer-Arrays nach Anspruch 3; und einen Prozessor (120), der angepasst ist, um Ultraschallsignale, die von dem Ultraschall-Transducer-Array (110) oder einem ausgewählten Ultraschall-Transducer-Array aus dem Satz von Transducer-Arrays erhoben werden, zu empfangen und zu verarbeiten.

## Revendications

1. Réseau de transducteurs à ultrasons (T) comportant plusieurs éléments transducteurs (TE) ayant chacun un champ de vision respectif (FOV-1 - FOV-5), le réseau de transducteurs à ultrasons (T) pouvant être positionné autour d'un objet ayant une surface incurvée (S) entraînant une convergence des champs de vision respectifs des éléments transducteurs (TE), dans lequel les éléments transducteurs (TE) sont disposés, ou peuvent être disposés, sur le réseau de transducteurs à ultrasons (T) de manière opposée à la courbure de la surface incurvée (S) afin de contrer la convergence des champs de vision respectifs, dans lequel les orientations respectives des éléments transducteurs (TE) peuvent être ajustées, et **caractérisé par le fait que** les éléments transducteurs (TE) sont disposés dans un milieu qui peut être amené à changer d'état, de liquide à solide, fixant ainsi les orientations respectives.

2. Réseau de transducteurs à ultrasons (T) selon la revendication 1, dans lequel les éléments transducteurs (TE) sont disposés dans le réseau de transducteurs à ultrasons (T) sous des angles d'inclinaison respectifs opposés aux angles auxquels les éléments transducteurs (TE) seront positionnés lorsque le réseau de transducteurs (T) est placé autour de l'objet.

3. Ensemble de réseaux de transducteurs à ultrasons (T) selon la revendication 2, l'ensemble comprenant:
un premier réseau de transducteurs à ultrasons (T) comportant des premiers éléments transducteurs (TE) disposés dans le premier réseau de transducteurs à ultrasons (T) sous des premiers angles d'inclinaison respectifs; et
un deuxième réseau de transducteurs à ultrasons (T) comportant des éléments de transducteurs (TE) disposés dans le deuxième réseau de transducteurs à ultrasons (T) selon des angles d'inclinaison respectifs, chacun des deuxièmes angles d'inclinaison respectifs dépassant un angle d'inclinaison correspondant parmi les premiers angles d'inclinaison respectifs.

4. Système à ultrasons (100), le système comprenant:
un réseau de transducteurs à ultrasons (110) selon l'une des revendications précédentes 1-2, ou un ensemble de réseaux de transducteurs à ultrasons selon la revendication 3; et
un processeur (120) adapté pour recevoir et traiter les signaux ultrasonores acquis par le réseau de transducteurs à ultrasons (110) ou par un réseau de transducteurs à ultrasons sélectionné dans l'ensemble de réseaux de transducteurs.
